(19) Europäisches Patentamt
European Patent Office
Office européen des brevets

(11) Veröffentlichungsnummer: **0 402 734 A2**

(12) # EUROPÄISCHE PATENTANMELDUNG

(21) Anmeldenummer: **90110574.2**

(51) Int. Cl.5: **A61K 31/55**

(22) Anmeldetag: **05.06.90**

(30) Priorität: **10.06.89 DE 3919076**

(43) Veröffentlichungstag der Anmeldung:
**19.12.90 Patentblatt 90/51**

(84) Benannte Vertragsstaaten:
**AT BE CH DE DK ES FR GB GR IT LI LU NL SE**

(71) Anmelder: **Dr. Karl Thomae GmbH**
**Postfach 1755**
**D-7950 Biberach 1(DE)**

(72) Erfinder: **Engel, Wolfhard, Dr. Dipl.-Chem.**
**Mozartstrasse 13**
**D-7950 Biberach 1(DE)**
Erfinder: **Mayer, Norbert, Dr.**
**Friedrich-Ebert-Strasse 66**
**D-7950 Biberach 1(DE)**
Erfinder: **Doods, Henri, Dr.**
**Hornsteinweg 7**
**D-7951 Warthausen(DE)**
Erfinder: **Eberlein, Wolfgang, Dr. Dipl.-Chem.**
**Obere Au 6**
**D-7950 Biberach 1(DE)**
Erfinder: **Mihm, Gerhard, Dr. Dipl.-Chem.**
**Nickeleshalde 5/1**
**D-7950 Biberach 1(DE)**
Erfinder: **Rudolf, Klaus, Dr.**
**Marktplatz 38**
**D-7950 Biberach 1(DE)**

(54) **Mittel zur Behandlung von Erkrankungen des Zentralnervensystems und zur Förderung der cerebralen Durchblutung.**

(57) Kondensierte Diazepinone der allgemeinen Formeln

EP 0 402 734 A2

Xerox Copy Centre

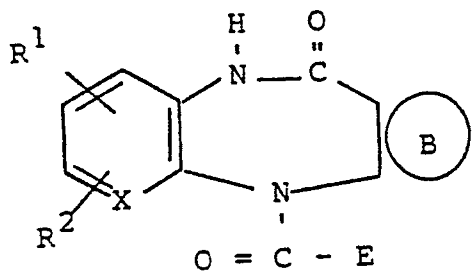

(Ia)

und

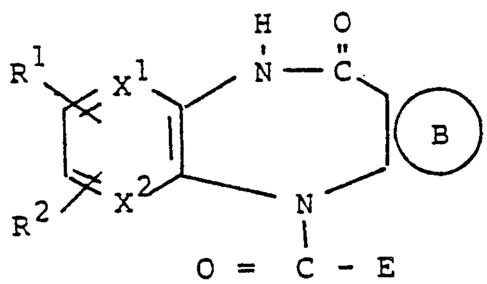

(Ib)

laßen sich zur Therapie von Erkrankungen des Zentralnervensystems insbesondere zur Behandlung des Morbus Alzheimer und Morbus Parkinson, verwenden. Sie steigern die cerebrale Durchblutung und sind deshalb auch in der Geriatrie und zur Bekämpfung von Migräne einsetzbar, darüberhinaus zeigen sie auch eine antiemetische Wirkung.

## Mittel zur Behandlung von Erkrankungen des Zentralnervensystems und zur Förderung der cerebralen Durchblutung

Die Erfindung betrifft die Verwendung eines Mittels zur Behandlung von Erkrankungen des Zentralnervensystems und zur Förderung der cerebralen Durchblutung.

Aus EP-A-0 039 519 und 0 057 428 sowie aus US-A 3.660.380; 3.691.159; 4.213.984; 4.213.985; 4.210.648, 4.410.527; 4.424.225; 4.424.222 und 4.424.226 sind bereits kondensierte Diazepinone mit ulcushemmenden und magensaftsekretionshemmenden Eigenschaften bekannt.

In EP-A-0 156 191, EP-A-0 213 293, EP-A-0 254 955, EP-A-0 273 239, EP-A-0 306 698, EP-A-0 312 895, DE-A-38 02 334, DE-A-38 19 444, DE-A-38 20 346, DE-A-38 20 345, ist beschrieben, daß durch Einführung neuartiger Aminoacylreste gegenüber den Verbindungen der obengenannten Publikationen völlig andersartige, wertvolle pharmakologische Eigenschaften induziert werden können; diese Verbindungen besitzen günstige Effekte auf die Herzfrequenz, sie sind angesichts fehlender magensäuresekretionshemmender, salivationshemmender und mydriatischer Einflüsse als vagale Schrittmacher zur Behandlung von Bradycardien und Bradyarrhythmien in der Human- und Veterinärmedizin geeignet, ein Teil der Verbindungen zeigt auch spasmolytische Eigenschaften auf periphere Organe, insbesondere Colon und Blase. Auch die in der DE-A-38 00 986.2 beschriebenen Verbindungen dienen als vagale Schrittmacher zur Behandlung von Bradycardien und Bradyarrhythmien.

Es wurde nun überraschenderweise gefunden, daß die in den vorgenannten Publikationen als vagale Schrittmacher zur Behandlung von Bradycardien und Bradyarrhythmien beschriebenen Verbindungen überraschenderweise noch gänzlich andersartige Wirkungen ausüben. Auf Grund ihrer antiemetischen Eigenschaften eignet sich ein Teil dieser Verbindungen auch zur Vorbeugung gegen Reise- und Seekrankheiten und angesichts günstiger Effekte auf die cerebrale Durchblutung zur Anwendung in der Geriatrie und bei Migräne. Vor allem aber zeigt eine Reihe dieser Verbindungen infolge ihrer hohen Lipophilie eine gute ZNS-Gängigkeit und läßt sich zur Therapie von Erkrankungen des Zentralnervensystems, insbesondere des Morbus Alzheimer und des Morbus Parkinson, einsetzen.

Es handelt sich im einzelnen um folgende Verbindungen der allgemeinen Formel Ia

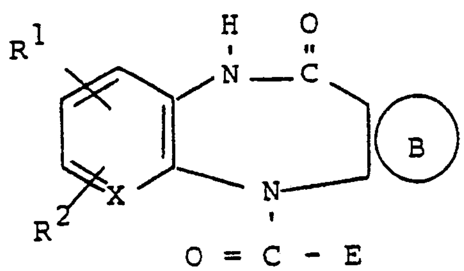

(Ia)

in der ] Ⓑ einen der zweiwertigen Reste

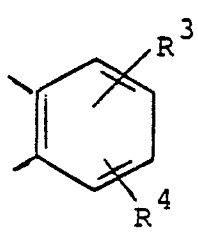

(S)

,

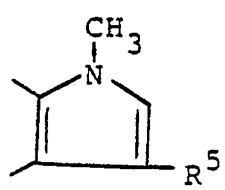

(T)

,

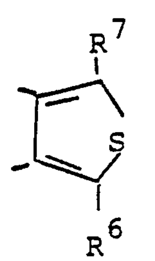

(U)

,

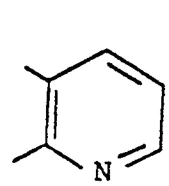

(V)

bedeutet und

X, $R^1$ bis $R^7$ und E die folgenden Bedeutungen besitzen:

a.) E ist eine Gruppe der Formel II

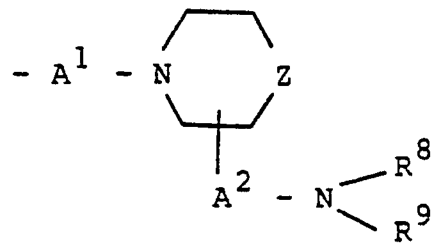

(II)

sofern X die =CH-Gruppe ist und ] Ⓑ für einen der zweiwertigen Reste (S), (T) oder (U) steht oder, im Falle, daß ] Ⓑ den ortho-Phenylenrest darstellt, X auch ein Stickstoffatom bedeutet,
wobei in den Formeln Ia und II
A' einen Alkylenrest mit 1 bis 2 Kohlenstoffatomen, $A^2$ entweder einen Alkylenrest mit 1 oder 2 Kohlenstoffatomen, sofern er sich in der 2-Stellung zum Stickstoff des gesättigten, heterocyclischen Ringes befindet oder, wenn er sich in der 3- oder 4-Stellung befindet, eine Einfachbindung oder die Methylengruppe bedeuten, und
Z entweder eine Einfachbindung oder ein Sauerstoffatom oder die Methylen- oder 1,2-Ethylengruppe darstellt,
R' eine Alkylgruppe mit 1 bis 4 C-Atomen, ein Chloratom oder ein Wasserstoffatom,
$R^2$ ein Wasserstoffatom oder eine Methylgruppe,
$R^3$ und $R^4$ jeweils ein Wasserstoffatom, ein Fluor-, Chlor- oder Bromatom oder eine Alkylgruppe mit 1 bis 4 C-Atomen bedeuten,
$R^5$ ein Wasserstoff- oder Chloratom oder die Methylgruppe,
$R^6$ ein Wasserstoff- oder Chloratom oder einen Alkylrest mit 1 bis 4 C-Atomen darstellen,
$R^7$ ein Wasserstoffatom oder einen Alkylrest mit 1 bis 4 C-Atomen,
$R^8$ einen verzweigten oder unverzweigten Alkylrest mit 1 bis 3 C-Atomen,
$R^9$ einen verzweigten oder unverzweigten Alkylrest mit 1 bis 7 C-Atomen darstellen, wobei gegebenenfalls der Alkylrest noch an seinem 2. bis 7. Kohlenstoffatom durch eine Hydroxygruppe substituiert sein kann, wobei $R^9$ aber auch einen Cycloalkyl- oder Cycloalkylmethylrest mit 3 bis 7 C-Atomen im Ring, der gegebenenfalls noch durch eine Hydroxygruppe substituiert sein kann, bedeuten kann, und im Falle, daß $R^1$ bis $R^4$ nicht alle gleichzeitig Wasserstoffatome bedeuten sondern wenigstens einer dieser Reste eine der anderen oben angegebenen Bedeutungen einnimmt, $R^8$ und $R^9$ lediglich gleiche oder verschiedene Alkylreste mit 1 bis 6 C-Atomen, A' die Methylengruppe und X das Stickstoffatom bedeuten; wobei in der Gruppe E $R^8$ und $R^9$ aber auch zusammen mit dem dazwischenliegenden Stickstoffatom einen 4- bis 7-gliedrigen gesättigten, monocyclischen heterocyclischen Ring bilden können, der gegebenenfalls durch ein Sauerstoffatom oder durch die $N-CH_3$-Gruppe unterbrochen sein kann,

b.) E ist eine Gruppe der Formel III

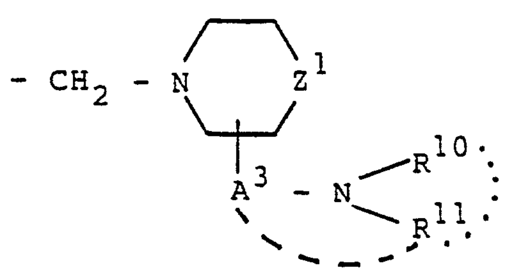

(III)

sofern in der Formel Ia
X eine =CH-Gruppe oder ein Stickstoffatom,
$R^1$ und $R^2$ Wasserstoffatome und
] Ⓑ die zweiwertigen Reste (S), (T), (U) und (V) mit den oben angegebenen Bedeutungen für die Reste $R^3$ bis $R^7$ darstellen,
wobei in der Formel III
$A^3$ einen geradkettigen oder verzweigten, gesättigten Alkylenrest mit 3 bis 7 Kohlenstoffatomen, der auch

durch ein Sauerstoff- oder Schwefelatom oder die Gruppe >$NR^{12}$, in der $R^{12}$ für eine Alkylgruppe mit 1 bis 3 C-Atomen steht, unterbrochen sein kann, darstellt,
$Z^1$ eine Einfachbindung, ein Sauerstoff- oder Schwefelatom, die Methylen- oder 1,2-Ethylengruppe bedeutet,
$R^{10}$ einen verzweigten oder unverzweigten Alkylrest mit 1 bis 7 Kohlenstoffatomen, einen Cycloalkyl- oder (Cycloalkyl)alkylrest mit insgesamt bis zu 8 Kohlenstoffatomen, eine gegebenenfalls mit einem Fluor-, Chlor- oder Bromatom und/oder einer Methyl-, Methoxy- oder Trifluormethylgruppe substituierte Aralkylgruppe mit bis zu 9 Kohlenstoffatomen, eine aliphatische Acylgruppe mit bis zu 7 Kohlenstoffatomen oder eine gegebenenfalls durch ein Fluor-, Chlor- oder Bromatom und/oder eine Methyl-, Methoxy- oder Trifluormethylgruppe substituierte Benzoylgruppe bedeutet,
$R^{11}$ eine verzweigte oder unverzweigte Alkylgruppe mit 1 bis 6 Kohlenstoffatomen darstellt wobei $R^{11}$ aber auch ein Wasserstoffatom sein kann, wenn $R^{10}$ die vorerwähnte aliphatische Acylgruppe oder gegebenenfalls substituierte Benzoylgruppe darstellt,
$R^{10}$ und $R^{11}$ aber auch zusammen mit dem dazwischenliegenden Stickstoffatom einen gesättigten, monocyclischen 5-, 6- oder 7-gliedrigen Ring bilden können, der gegebenenfalls noch durch eine Aminocarbonyl-, Dimethylaminocarbonyl- oder Diethylaminocarbonylgruppe substituiert und/oder durch ein Sauerstoffatom unterbrochen sein kann,
$R^{11}$ aber auch mit einem Kohlenstoffatom der $A^3$-Kette in der Weise verknüpft sein kann, daß zusammen mit der Gruppe $NR^{12}$ ein gesättigter 5-, 6- oder 7-gliedriger heterocyclischer Ring entsteht,

c.) E ist eine Gruppe der Formel IV

$-A^4 - CH_2 - C \equiv C - CH_2 - A^5$ (IV)

sofern in der Formel Ia
X ein Stickstoffatom, ]Ⓑ den ortho-Phenylenrest und $R^1$ bis $R^4$ Wasserstoffatome bedeuten wobei in der Formel IV
$A^4$ die Gruppen H- $\overset{|}{\underset{|}{C}}$ $-R^{13}$, $\overset{|}{\underset{|}{N}}$ $-R^{13}$ oder -0-, und
$A^5$ die Gruppen

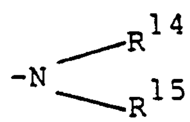 , 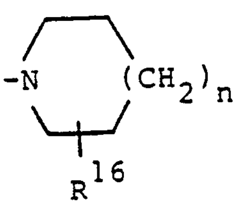 oder 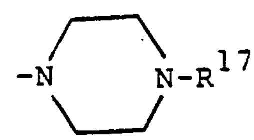

bedeuten, worin
$R^{13}$ ein Wasserstoffatom oder eine Alkylgruppe mit 1 bis 3 Kohlenstoffatomen,
$R^{14}$ und $R^{15}$, die gleich oder voneinander verschieden sein können, ein Wasserstoffatom, einen Alkylrest mit 1 bis 3 Kohlenstoffatomen, einen Phenylalkylrest mit insgesamt 7 bis 9 Kohlenstoffatomen, einen gegebenenfalls durch eine Hydroxygruppe substituierten 5- bis 7-gliedrigen Cycloalkylrest,
n die Zahlen 0, 1 oder 2,
$R^{16}$ ein Wasserstoffatom, eine Hydroxygruppe, eine Alkylgruppe mit 1 bis 3 Kohlenstoffatomen oder eine Gruppe der Formel

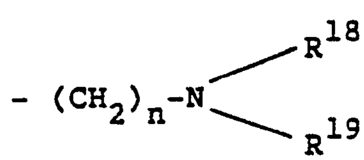 ,

worin n wie oben definiert ist und $R^{18}$ und $R^{19}$ einen Alkylrest mit 1 bis 3 Kohlenstoffatomen bedeuten und
$R^{17}$ eine gerade oder verzweigte Alkylgruppe mit 1 bis 3 Kohlenstoffatomen oder eine Phenylalkylgruppe mit insgesamt 7 bis 9 Kohlenstoffatomen darstellen,

d.) E ist eine Gruppe der Formel V

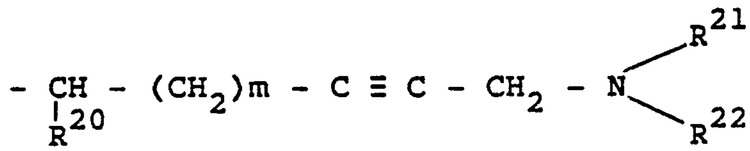

(V)

sofern in der Formel Ia X eine =CH-Gruppe und ] Ⓑ den zweiwertigen Rest (U) bedeuten, worin $R^6$ und $R^7$ wie oben definiert sind, $R^6$ aber zusätzlich noch ein Fluor- oder Bromatom sein kann und $R^1$ und $R^2$ Wasserstoffatome sind, wobei in der Formel V

$R^{20}$ ein Wasserstoffatom oder eine Alkylgruppe mit 1 bis 3 Kohlenstoffatomen,

$R^{21}$ ein Wasserstoffatom oder eine Alkylgruppe mit 1 bis 4 Kohlenstoffatomen,

$R^{22}$ eine gegebenenfalls durch eine Phenylgruppe substituierte Alkylgruppe mit 1 bis 3 Kohlenstoffatomen oder eine gegebenenfalls durch eine Hydroxygruppe substituierte 5-bis 7-gliedrige Cycloalkylgruppe oder $R^{21}$ und $R^{22}$ zusammen mit dem dazwischenliegenden Stickstoffatom einen 5- bis 7-gliedrigen gegebenenfalls durch einen Dialkylaminoalkylrest, eine Hydroxygruppe oder einen Alkylrest substituierten gesättigten monocyclischen Ring oder den 1-Piperazinylrest, der in 4-Stellung durch eine Alkyl-, Phenylalkyl- oder Phenylgruppe substituiert ist, wobei jeweils die vorstehend erwähnten Alkylteile 1 bis 3 Kohlenstoffatome enthalten können, und

m die Zahlen 1, 2 oder 3 darstellen,

e.) E ist eine Gruppe der Formel VI

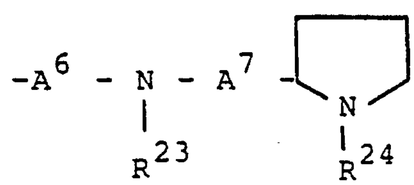

(VI)

sofern in der Formel Ia X eine =CH-Gruppe oder ein Stickstoffatom, ] Ⓑ die zweiwertigen Reste (S), (T), (U), (V) und zusätzlich den zweiwertigen Rest

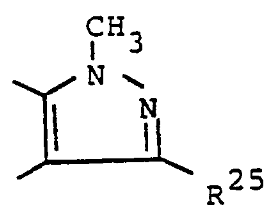

(W)

darstellen, in welchen die Reste $R^1$ bis $R^7$ wie oben definiert sind, aber $R^6$ noch zusätzlich ein Fluor- oder Bromatom sein kann, und $R^{25}$ ein Wasserstoffatom oder die Methylgruppe bedeutet,

wobei in der Formel VI

$A^6$ und $A^7$ geradkettige, gesättigte Alkylenreste mit 1 bis 4 Kohlenstoffatomen,

$R^{23}$ und $R^{24}$ Wasserstoffatome oder verzweigte oder unverzweigte Alkylreste mit 1 bis 4 Kohlenstoffatomen oder Cycloalkylreste mit 4 bis 7 Kohlenstoffatomen, die gegebenenfalls noch durch eine Hydroxygruppe substituiert sein können, bedeuten,

wobei in dem Fall, daß ] Ⓑ den zweiwertigen Rest (T) darstellt und $R^5$ ein Wasserstoffatom ist, $R^1$ kein Chloratom sein kann und in dem Fall, daß ] Ⓑ den zweiwertigen Rest (V) bedeutet, X kein Stickstoffatom ist,

f.) E ist eine Gruppe der Formel VII

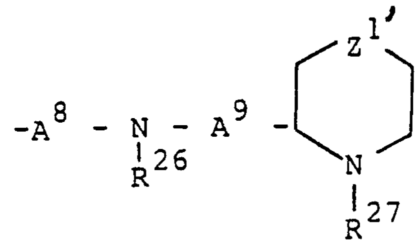

(VII)

sofern in der Formel Ia

X eine =CH-Gruppe, ein Stickstoffatom oder, zusätzlich, eine =C-Cl-Gruppe, ] Ⓑ einen der zweiwertigen Reste (S), (T), (U) oder (V) bedeuten, wobei ] Ⓑ nur dann den zweiwertigen Rest (V) bedeutet, wenn X die =CH-Gruppe oder =C-Cl-Grupppe darstellt, und wobei $R^1$ und $R^2$ Wasserstoffatome bedeuten und $R^3$ bis $R^7$ wie oben definiert sind, und $R^6$ zusätzlich noch die Bedeutung eines Fluor- oder Bromatoms haben kann,

wobei in der Formel VII

$A^8$, und $A^9$ geradkettige oder verzweigte gesättigte Alkylenreste mit 1 bis 4 Kohlenstoffatomen,

$Z^1$ ein Sauerstoffatom oder eine Alkylenkette mit 1 bis 3 Kohlenstoffatomen

$R^{26}$ einen verzweigten oder unverzweigten Alkylrest mit 1 bis 7 Kohlenstoffatomen, einen Cycloalkylrest oder (Cycloalkyl)alkylrest mit insgesamt bis zu 8 Kohlenstoffatomen oder ein Wasserstoffatom und

$R^{27}$ einen verzweigten oder unverzweigten Alkylrest mit 1 bis 6 Kohlenstoffatomen oder eine Cycloalkylgruppe mit bis zu 7 Kohlenstoffatomen bedeuten,

und deren Isomere und physiologisch verträglichen Salze mit anorganischen oder organischen Säuren.

Aber auch kondensierte Diazepinone der allgemeinen Formel Ib

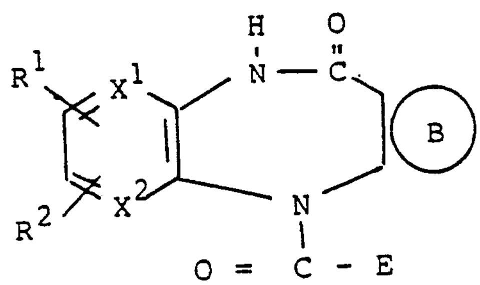

(Ib)

deren Isomere und physiologisch verträgliche Salze mit anorganischen oder organischen Säuren zeigen dieselben kurativen Wirkungen.

In der Formel Ib sind die Substituenten wie folgt definiert

] Ⓑ bedeutet einen der zweiwertigen Reste

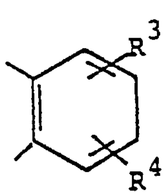 , 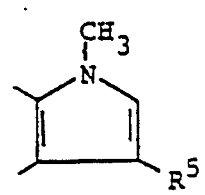 , 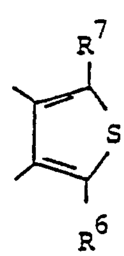 , 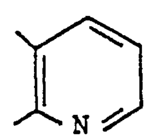 oder 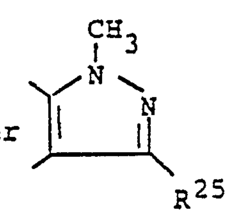

(S) (T) (U) (V) (W)

$X^1$ und $X^2$ eine =CH-Gruppe oder, sofern ] Ⓑ die Bedeutungen der oben genannten Reste (S), (U) oder (W) annimmt, können beide oder nur $X^1$ oder nur $X^2$ auch ein Stickstoffatom sein,

$R^1$ bis $R^7$ und $R^{25}$ sind wie im Falle der Formel Ia definiert, wobei $R^6$ zusätzlich auch ein Fluor- oder Bromatom sein kann,

und E bedeutet

a.) eine Gruppe der Formel VIII

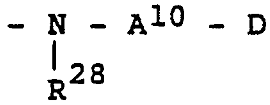

(VIII)

in der

$R^{28}$ ein Wasserstoffatom oder eine Methylgruppe,

D die Gruppen

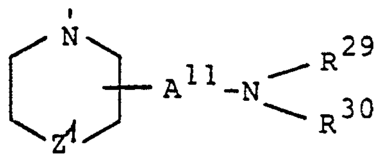 , 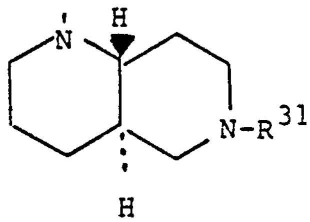 , 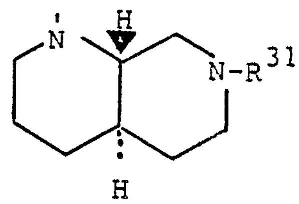 ,

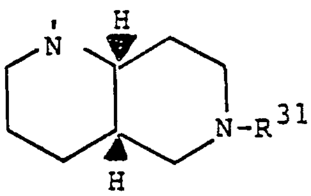 und 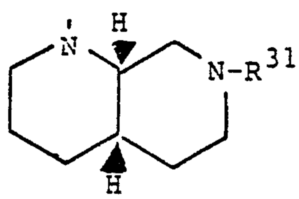

und

$A^{10}$ einen geradkettigen oder verzweigten Alkylenrest mit 2 bis 7 Kohlenstoffatomen darstellen, wobei in den Gruppen D

$Z^1$ wie oben für die Formel Ia definiert ist,

$A^{11}$ einen geradkettigen oder verzweigten gesättigten Alkylenrest mit 1 bis 5 Kohlenstoffatomen oder, wenn $A^{11}$ sich in 3-Stellung zum Stickstoff des gesättigten heterocyclischen Ringes befindet, auch eine Einfachbindung darstellt,

$R^{29}$ einen verzweigten oder unverzweigten Alkylrest mit 1 bis 4 Kohlenstoffatomen,

$R^{30}$ einen verzweigten oder unverzweigten Alkylrest mit 1 bis 7 Kohlenstoffatomen, der gegebenenfalls noch an seinem 2. bis 7. Kohlenstoffatom durch eine Hydroxygruppe substituiert sein kann, des weiteren einen Cycloalkyl- oder einen Cycloalkylmethylrest mit 3 bis 7 Kohlenstoffatomen im Ring, wobei der Cycloalkylring gegebenenfalls noch durch eine Hydroxygruppe substituiert sein kann, bedeuten, wobei $R^{29}$ und $R^{30}$ aber auch zusammen mit dem dazwischenliegenden Stickstoffatom einen 4- bis 7-gliedrigen gesättigten, monocyclischen, heterocyclischen Ring bilden können, der gegebenenfalls durch ein Sauerstoffatom oder durch die Gruppe >N-$CH_3$ unterbrochen sein kann, und

$R^{31}$ einen verzweigten oder unverzweigten Alkylrest mit 1 bis 6 Kohlenstoffatomen darstellt,

b.) eine Gruppe der Formel IX

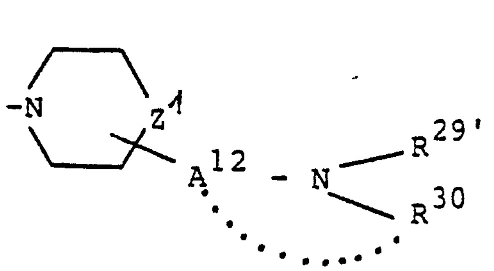

(IX)

worin
$Z^1$ wie oben definiert ist,
$A^{12}$ einen geradkettigen oder verzweigten gesättigten Alkylenrest mit 2 bis 7 Kohlenstoffatomen, der auch durch ein Sauerstoff- oder Schwefelatom oder durch die Methylimino-oder Ethyliminogruppe unterbrochen sein kann, bedeutet und
$R^{29}$ und $R^{30}$ die vorstehend genannten Bedeutungen innehaben, wobei sich $R^{29'}$ von $R^{29}$ dadurch unterscheidet, daß $R^{29}$ zusätzlich noch die Bedeutung einer Benzylgruppe einnehmen kann; $R^{30}$ kann aber zusätzlich auch mit $A^{12}$ über eine Alkylenbrücke in der Weise verknüpft sein, daß zusammen mit der Gruppe $-NR^{29}$ ein gesättigtes, 5-, 6- oder 7-gliedriges heterocyclisches Ringsystem entsteht,

c.) eine Gruppe der Formel X

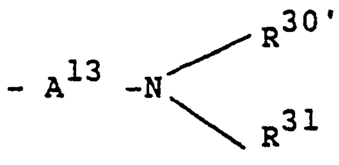

(X)

in der
$R^{30}$ eine Alkylgruppe mit 1 bis 3 Kohlenstoffatomen,
$R^{31}$ eine gegebenenfalls ab dem 2. Kohlenstoffatom durch eine Hydroxygruppe substituierte geradkettige oder verzweigte Alkylgruppe mit 1 bis 8 Kohlenstoffatomen, eine Cycloalkyl- oder Cycloalkylmethylgruppe, in welcher der Cycloalkylteil jeweils 3 bis 8 Kohlenstoffatome enthalten kann und durch eine Methyl- oder Hydroxygruppe substituiert sein kann, eine Phenylalkylgruppe, in welcher der Alkylteil 1 bis 3 Kohlenstoffatome enthalten kann und der Phenylrest durch ein Halogenatom, eine Methyl-, Methoxy- oder Trifluormethylgruppe mono- oder disubstituiert sein kann, wobei die Substituenten des Phenylkerns gleich oder verschieden sein können bedeuten, oder
$R^{30}$ und $R^{31}$ zusammen mit dem dazwischenliegenden Stickstoffatom einen 6- bis 8-gliedrigen gesättigten monocyclischen Ring bilden, in welchem eine Methylengruppe mit der Maßgabe, daß mindestens zwei Kohlenstoffatome zwischen dieser Methylengruppe und dem bereits vorhandenen Stickstoffatom liegen, durch ein Sauerstoffatom oder durch eine gegebenenfalls durch eine Alkyl-, Phenylalkyl- oder Phenylgruppe substituierte Iminogruppe, in welcher der Alkylteil jeweils 1 bis 3 Kohlenstoffatome enthalten kann und der Phenylkern jeweils durch ein Halogenatom, eine Methyl-, Methoxy- oder Trifluormethylgruppe mono- oder disubstituiert sein kann, wobei die Substituenten gleich oder verschieden sein können, ersetzt sein kann,
$A^{13}$ eine gegebenenfalls eine Doppelbindung enthaltende Alkylengruppe mit 5 bis 8 Kohlenstoffatomen darstellt mit der Maßgabe, daß
mindestens 5 Kohlenstoffatome zwischen der semicyclischen Carbonylgruppe und dem Stickstoffatom der

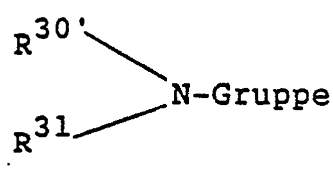

liegen,

d.) eine Gruppe der Formel XI

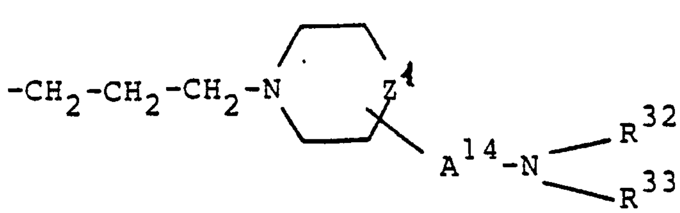

(XI)

worin $Z^1$ wie oben definiert ist,
$A^{14}$ einen geradkettigen, gesättigten Alkylenrest mit 1 bis 6 Kohlenstoffatomen,
$R^{32}$ und $R^{33}$ gleiche oder voneinander verschiedene Alkylreste mit bis zu 6 Kohlenstoffatomen bedeuten, die zusammen mit dem dazwischenliegenden Stickstoffatom einen 4- bis 7-gliedrigen, gesättigten, monocyclischen, heteroaliphatischen Ring, der gegebenenfalls durch ein Stickstoffatom oder durch die >N-$CH_3$ Gruppe unterbrochen sein kann, bilden können.

Auch die diastereomeren und enantiomeren Formen und deren Salze mit anorganischen oder organischen Säuren der Formel Ib zeigen dieselben kurativen Wirkungen und eignen sich zu denselben Verwendungszwecken.

Die Herstellung von Verbindungen der allgemeinen Formel Ia wird in den folgenden Publikationen beschrieben: EP-A-0 156 191 (entspr. US-Patent Nr. 4.550.107), EP-A-0 254 955, EP-A-0 312 895, EP-A-0 213 293 (entspr. US-Patent 4.724.236), DE-A-38 00 986.2, DE-A-38 20 346 und DE-A-38 20 345, die Herstellung von Verbindungen der allgemeinen Formel Ib in den folgenden Publikationen: EP-A-0 273 239, EP-A-0 306 698, DE-A-38 02 334 und DE-A-38 19 444.

Die Verbindungen der allgemeinen Formeln Ia und Ib zeigen ganz allgemein vorteilhafte Effekte auf die cerebrale Durchblutung und eignen sich daher zur Anwendung in der Geriatrie und bei Migräne.

Die Stimulierung muscarinischer Rezeptoren in endothelgeschädigten Arterien, etwa in Coronar- oder Basilararterien, führt zu einer Konstriktion. Beispielsweise vermindert vagal freigesetztes Acetylcholin den coronaren Blutfluß in isolierten Rattenherzen, Methacholin bewirkt dosisabhängige Kontraktionen der Coronar- und Basilararterien im in-vitro-Experiment (K.J. van Charldorp, Dissertation "Characterisation of Muscarinic Receptors in the Vascular System", Amsterdam 1988; K.J. van Charldorp, D. Davidesko und P.A. van Zwieten, Eur. J. Pharmacol. 150, 197-199 (1988); K.J. van Charldorp und P.A. van Zwieten, Naunyn Schmiedeberg's Arch. Pharmacol. 339, 403-408 (1989)).

Die genannten Untersuchungen geben klare Hinweise darauf, daß die cholinerge Stimulation vasculärer muscarinischer Rezeptoren von pathophysiologischer Bedeutung ist, beispielsweise bei Patienten, die an Prinzmetal-Angina leiden oder cerebralsklerotisch geschädigt sind. Hemmung der in Hirngefäßen nachweisbaren muscarinischen Rezeptoren führt also zu einer Vermeidung der Konstriktionen und zu einer Verbesserung bzw. Normalisierung der arteriosklerotisch bedingten Störungen der Cerebraldurchblutungen. Die Verbindungen der vorliegenden Anmeldung hemmen die in Cerebralgefäßen, beispielsweise in der Pia mater, nachweisbaren muscarinischen Rezeptoren bereits bei physiologisch erreichbaren Konzentrationen bzw. Plasmaspiegeln und sind zur Entwicklung von Therapeutica zur Behandlung arteriosklerotisch bedingter Durchblutungsstörungen der ZNS geeignet.

Eine Reihe der Verbindungen der allgemeinen Formeln Ia und Ib zeigen infolge hoher Lipophilie eine gute ZNS-Gängigkeit und eignen sich daher zusätzlich auch zur Therapie von Erkrankungen des Zentralnervensystems, insbesondere des Morbus Alzheimer und des Morbus Parkinson. Beim Morbus Alzheimer beeinflussen die Verbindungen die autoregulatorische Funktion präsynaptischer Muskarinrezeptoren auf die Freisetzung von Acetylcholin und führen dadurch zu einer Verstärkung des Impulsmusters der noch vorhandenen cholinergischen Fasern, beim Morbus Parkinson besteht der Vorteil des Einsatzes der Verbindungen der allgemeinen Formel Ia und Ib an Stelle der bisher üblichen nichtselektiven Antimuskarinika in dem Fehlen nichttolerabler peripherer und zentraler atropinartiger Nebenwirkungen.

Bei seniler Demenz vom Alzheimer-Typ führt Degeneration cholinerger Neuronen, insbesondere in hippocampalen und corticalen Projektionen, zu verminderter Freisetzung des Neurotransmitters Acetylcholin. Die Blockade der präsynaptischen Autorezeptoren unterbricht nun den negativen Rückkopplungsmechanismus, den der Neurotransmitter an den noch intakten Neuronen ausübt, und bewirkt daher einen gesteigerten Acetylcholin-Release und - damit verknüpft - eine Stimulierung der postsynaptischen Rezeptoren (D.C. Mash, D L. Flynn und L.T. Potter, Science 228, 115-117 (1985); E.K. Perry u. a., Can. J. Neurol. Sci. 13, 521-527 (1986); M. Sarter u. a., TINS 11, 13-17 (1988)).

Zum Nachweis der günstigen Effekte auf die cerebrale Durchblutung und den vermehrten Acetylcholin-Release wurden die folgenden Versuche durchgeführt:

A. Bindung verschiedener muskarinischer Antagonisten an Rezeptoren der Pia mater des Rindes

Die Pia-Gefäße von Rinder-Hirnen, erhalten vom lokalen Schlachthof, wurden präpariert und von Blutresten gereinigt. Je 0,5 g-Portionen wurden bei -80°C bis zur weiteren Verwendung gelagert. Zur Homogenatgewinnung wurden 0,5 g Pia-Gefäße mittels eines Gewebehackers zerkleinert, in 60 ml HEPES-Puffer (20 mM HEPES, 10 mM $MgCl_2$, 100 mM NaCl, pH 7,50) aufgenommen, mit einem Ultra-Turrax homogenisiert und 20 min. bei 0°C mit 48000 x g zentrifugiert. Das Pellet wurde erneut mit einem Ultra-Turrax homogenisiert, durch Mull filtriert und mit HEPES-Puffer auf eine Verdünnung von 1:140 bezogen auf das Feuchtgewicht der Gefäße verdünnt.

0,3 nM [$^3$H]NMS ($^3$H-N-Methylscopolamin) und steigende Konzentrationen des Antagonisten wurden mit dem Homogenat in einem Gesamtvolumen von 0,52 ml für 45 min. bei Raumtemperatur inkubiert. Der Inkubationsansatz wurde mit Hilfe eines Scatron Cell Harvesters über Glasfaser-Filtermatten filtriert und zweimal mit eiskalter physiologischer Kochsalzlösung gewaschen. Die Filter-gebundene Radioaktivität wurde in einem Flüssigkeitsszintillationszähler bestimmt. Als unspezifische Bindung wurde die gebundene Radioaktivität in Gegenwart von $10^{-6}$ M (-)-Chinuclidinyl-benzilat definiert.

B. Transmitter Release Studien

Die Versuche zur in-vitro Freisetzung von Acetylcholin aus hippocampalen Gewebeschnitten wurden in Analogie zu Anord nungen durchgeführt, die für die Freisetzung von 5-HT aus Hypothalamus- bzw. Hippocampus-Gewebe beschrieben sind (vgl. M.H. Richards, Naunyn Schmiedeberg's Arch. Pharmacol. 329, 359-366 (1985) und T.J. Feuerstein, G. Hertting und R. Jackisch, Europ. J. Pharmacol. 107, 233-242 (1985)).

Männliche Ratten (Stamm Chbb:Thom, Gewicht 200 bis 300 g) wurden ohne Vorbehandlung dekapitiert; das gesamte Gehirn wurde sofort entnommen und unter Eiskühlung zerlegt. Gemäß den oben beschriebenen Verfahren wurde der Hippocampus in 0,4 mm dicke Schnitte mit Hilfe eines McIlwain-Gewebehackers geschnitten. Die Gewebeschnitte wurden 30 Minuten lang bei 37° C vorincubiert in Gegenwart von 0,05 mM $^3$H-Cholin in Krebs-Henseleit Puffer der folgenden Zusammensetzung: 120 mM NaCl, 4,8 mM KCl, 1,3 mM $CaCl_2$, 1,2 mM $KH_2PO_4$, 1,2 mM $MgSO_4$ und 25 mM $NaHCO_3$. Der Puffer enthielt zusätzlich 2 g Glucose und 100 mg EDTA. Der Puffer wurde mit Natronlauge auf einen pH-Wert von 7,4 bei 20° C eingestellt. Alle Lösungen wurden kontinuierlich mit 95 % $O_2$ und 5 % $CO_2$ gesättigt. Die Gewebeschnitte wurden daraufhin gespült, jeder Gewebeschnitt wurde jeweils willkürlich auf 12 Superfusionseinheiten gegeben und mit der Pufferlösung bei 37° C bei einer Fließgeschwindigkeit von 1 ml/min überspült. Die Gewebeschnitte wurden jeweils von einem Polypropylennetzwerk zwischen zwei Platinelektroden gehalten. Nach 50-minütiger Überspülung wurden 4-Minuten-Fraktionen des Superfusats über eine gesamte Zeitdauer von 92 Minuten gesammelt. Die Gewebeschnitte wurden zweimal elektrisch stimuliert, und zwar 60 Minuten (S1) und 100 Minuten (S2) nach Beginn der Superfusion. Dabei wurden Rechteckimpulse von 2 ms Dauer und mit einer Frequenz von 3 Hz 100 Sekunden lang bei 5 V/cm und 24 mA mittels eines HSE Stimulators II der Firma Hugo Sachs Elektronik, Hugstetten, West-Deutschland, verabreicht. Die zu prüfenden Substanzen wurden dem Puffer ab 20 Minuten vor dem Zeitpunkt S2 zusammen mit dem Agonisten (Carbachol) beigefügt. Am Ende wurden die Gewebeschnitte aus den Kammern entfernt und zur Bestimmung des verbliebenen Tritiums solubilisiert.

Die Filterscheiben mit der Radioaktivität, die an die Rezeptoren oder an die Membranen gebunden ist, zusammen mit den Synaptosomen wurden in Minivials unter Hinzufügen von 3 ml eines Scintillation-Cocktails (Quickszint 2000, Zinsser Analytic, Frankfurt, West-Deutschland) gegeben und eine Stunde lang langsam rotiert. Die Extrakte der MAO-Aktivitätsbestimmung (4 ml) wurden in Standard-Vials mit 10 ml dieses Cocktails übertragen. Die Radioaktivität wurde daraufhin mittels der üblichen Flüssig-Scintillations-Messung bestimmt mit externer Standardisierung in Beckman LS 3800 oder LS 7800 Instrumenten. Die Ergebnisse sind in der Tabelle II enthalten.

C. Bindungsstudien an muscarinischen Rezeptoren des Cortex

Als Organspender dienten männliche Sprague-Dawley-Ratten mit 180-220 g Körpergewicht. Nach Entnahme der Großhirnrinde wurden alle weiteren Schritte in eiskaltem Hepes-HCl-Puffer (pH 7,4; 100 m molar NaCl, 10 m molar $MgCl_2$) vorgenommen. Das Gesamtherz wurde mit einer Schere zerkleinert. Alle Organe wurden abschließend in einem Potter homogenisiert.

Für den Bindungstest wurden die Organhomogenate in folgender Weise verdünnt:

Gesamtherz 1: 400

Großhirnrinde 1:3000

Submandibularis 1: 400 (v:v)

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30° C. Die Inkubationsdauer be trug 45 Minuten. Als Radioligand wurde 0,3 n molar $^3$H-N-Methylscopolamin

($^3$H-NMS) verwendet. Die Inkubation wurde durch Zugabe von eiskaltem Puffer mit nachfolgender Vakuumfiltration beendet. Die Filter wurden mit kaltem Puffer gespült und ihre Radioaktivität bestimmt. Sie repräsentiert die Summe von spezifischer und unspezifischer Bindung von $^3$H-NMS. Der Anteil der unspezifischen Bindung wurde definiert als jene Radioaktivität, die in Anwesenheit von 1 $\mu$ molar Chinuclidinylbenzilat gebunden wurde. Es wurden immer Vierfachbestimmungen vorgenommen. Die $IC_{50}$-Werte der nichtmarkierten Testsubstanzen wurden graphisch bestimmt. Sie repräsentieren jene Konzentration der Testsubstanz, bei welcher die spezifische Bindung von $^3$H-NMS an die muscarinischen Rezeptoren in den verschiedenen Organen um 50 % gehemmt wurde. Die Ergebnisse sind aus der Tabelle II zu ersehen.

Nach den vorstehenden Angaben wurden beispielsweise die folgenden Verbindungen untersucht:

A = (+)-11-[[2-[(Diethylamino)methyl]-1-piperidinyl]acetyl]-5,11-dihydro-6H-pyrido[2,3-b][1,4]-benzodiazepin-6-on

B = 5,11-Dihydro-11-[1-oxo-6-(1-piperidinyl)-4-hexin-1-yl]-6H-pyrido[2,3-b][1,4]benzodiazepin-6-on

C = (±)-9-Chlor-11-[[2-[(diethylamino)methyl]-1-piperidinyl]acetyl]-5,11-dihydro-6H-pyrido[2,3-b][1,4]-benzodiazepin-6-on

D = 5,11-Dihydro-11-[[[2-[2-[(dipropylamino)methyl]-1-piperidinyl]ethyl]amino]carbonyl]-6H-pyrido[2,3-b]-[1,4]benzodiazepin-6-on-methansulfonat

E = 5,11-Dihydro-11-[[3-[3-(1-piperidinyl)-1-propyl]-1-piperidinyl]carbonyl]-6H-pyrido[2,3-b][1,4]-benzodiazepin-6-on

F = 4,9-Dihydro-3-methyl-4-[[4-[3-(1-piperidinyl)-1-propyl]-1-piperidinyl]acetyl]-10H-thieno[3,4-b][1,5]-benzodiazepin-10-on

G = 5,11-Dihydro-11-[1-oxo-6-(1-piperidinyl)-1-hexyl]-6H-pyrido[2,3-b][1,4]benzodiazepin-10-on

H = 4,9-Dihydro-3-methyl-4-[6-(hexahydro-1H-1-azepinyl)-1-oxo-4-hexin-1-yl]-10H-thieno[3,4-b][1,5]-benzodiazepin-10-on

I = 11-[4-[3-[(Diethylamino)methyl]-4-morpholinyl]-1-oxo-1-butyl]-5,11-dihydro-6H-pyrido[2,3-b][1,4]-benzodiazepin-6-on

K = 5,11-Dihydro-11-[[[2-(1-methyl-2-pyrrolidinyl)ethyl]methylamino]acetyl]-6H-pyrido[2,3-b][1,4]-benzodiazepin-6-on

L = 5,11-Dihydro-11-[[[2-(1-methyl-hexahydro-1H-2-azepinyl)ethyl]methylamino]acetyl]-6H-pyrido[2,3-b]-[1,4]benzodiazepin-6-on

Die folgenden Tabellen enthalten die dabei gefundenen Ergebnisse:

Tabelle I:

| Hemmung der $^3$H-NMS-Bindung an muscarinische Rezeptoren der Pia mater des Rindes | |
|---|---|
| Substanz | -log $IC_{50}$ (M) |
| A | 7,1 |
| B | 6,9 |
| C | 7,5 |
| D | 7,9 |
| E | 8,8 |
| F | 8,6 |
| H | 8,0 |
| I | 7,7 |
| K | 8,2 |
| L | 8,4 |

Tabelle II:

| Einfluß auf den Transmitter-Release im Vergleich zur Affinität zu muscarinischen Rezeptoren vom $M_1$-Subtyp | | | |
|---|---|---|---|
| Substanz | ACh-Release aus Hippocampus-Gewebe ($pA_2$) | Rezeptor-Bindungstests Cortex -log $IC_{50}$ (M) | Verhältnis von $IC_{50}$ (Cortex) zu $pA_2$ (ACh-Release) |
| A | 7,15 | 6,00 | 14 |
| B | 7,2 | 5,92 | 19 |
| C | 7,3 | 6,30 | 10 |
| D | 8,35 | 7,05 | 20 |
| E | 9,55 | 8,00 | 35 |
| F | 7,7 | 7,22 | 3 |
| G | 8,45 | 7,22 | 17 |
| H | 8,0 | 7,15 | 7 |
| I | 7,5 | 7,0 | 3 |
| K | 8,5 | 7,52 | 10 |
| L | 8,45 | 7,40 | 11 |

Die Tabelle I zeigt, daß die Verbindungen der allgemeinen Formeln Ia und Ib muscarinische Rezeptoren der Cerebralgefäße - als Beispiel wurden solche der Pia mater gewählt - bereits bei physiologisch erreichbaren Konzentrationen bzw. Plasmaspiegeln zu hemmen vermögen.

Die vorstehende Tabelle II beweist, daß die Verbindungen der allgemeinen Formeln Ia und Ib nicht nur bereits bei sehr niedrigen Konzentrationen die inhibitorischen Autorezeptoren in hippocampalem Gewebe blockieren und damit die Acetylcholin-Freisetzung erhöhen sondern darüber hinaus auch se lektiv sind, da sie erst bei merklich höheren Konzentrationen postsynaptische Rezeptoren vom $M_1$-Subtyp hemmen. Die Verbindungen ermöglichen also einen Ausgleich der für senile Demenz vom Alzheimer-Typ charakteristischen cholinergen Defizite und eignen sich zur Verwendung als Arzneimittel zur Bekämpfung des Morbus Alzheimer.

Gegenstand der Erfindung ist die Verwendung von Arzneimitteln zur Bekämpfung des Morbus Alzheimer und Morbus Parkinson, die ein oder mehrere kondensierte Diazepinone der allgemeinen Formeln Ia und Ib, deren physiologisch verträgliche Salze und/oder deren Isomere enthalten.

Die Verbindungen der allgemeinen Formeln Ia und Ib, deren Isomere und physiologisch verträglichen Salze lassen sich hierzu in an sich bekannter Weise in die üblichen pharmazeutischen Zubereitungsformen, z. B. in Lösungen, Suppositorien, Tabletten, Dragées, Kapseln oder Teezubereitungen einarbeiten. Die Tagesdosis liegt im allgemeinen zwischen 0,02 und 5 mg/kg, vorzugsweise 0,02 und 2,5 mg/kg, insbesondere 0,05 und 1,0 mg/kg Körpergewicht, die gegebenenfalls in Form mehrerer, vorzugsweise 1 bis 3 Einzelgaben, zur Erzielung der gewünschten Ergebnisse verabreicht wird.

Im folgenden wird die Herstellung pharmazeutischer Anwendungsformen anhand einiger Beispiele beschrieben:

Beispiel I

Tabletten mit 5 mg 11-[[2-[(Diethylamino)methyl]-1-piperidinyl]acetyl]-5,11-dihydro-6H-pyrido[2,3-b][1,4]-benzodiazepin-6-on

Zusammensetzung:

1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzukker und die restliche Kartoffelstärke werden gemischt und mit obigem Schleim durch ein Sieb der Maschenweite 1,5 mm granuliert. Das Granulat wird bei 45 °C getrocknet, nochmals durch obiges Sieb gerieben, mit Magnesiumstearat vermischt und zu Tabletten verpreßt.
Tablettengewicht: 220 mg
Stempel: 9 mm

Beispiel II

Dragées mit 5 mg 11-[[2-[(Diethylamino)methyl]-1-piperidinyl]acetyl]-5,11-dihydro-6H-pyrido[2,3-b][1,4]-benzodiazepin-6-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg

Beispiel III

Ampullen mit 1 mg 5,11-Dihydro-11-[1-oxo-6-(1-piperidinyl)-4-hexinyl]-6H-pyrido[2,3-b][1,4]benzodiazepin-6-on

Zusammensetzung:

1 Ampulle enthält:

| | | |
|---|---|---|
| Wirkstoff | | 10,0 mg |
| Natriumchlorid | | 8,0 mg |
| Dest. Wasser | ad | 1 ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.
Sterilisation: 20 Minuten bei 120 °C.

Beispiel IV

Suppositorien mit 20 mg 9-Chlor-11-[[2-[(diethylamino)methyl]-1-piperidinyl]acetyl]-5,11-dihydro-6H-pyrido-[2,3-b][1,4]benzodiazepin-6-on

Zusammensetzung:

1 Zäpfchen enthält:

| | |
|---|---|
| Wirkstoff | 20,0 mg |
| Zäpfchenmasse (z.B. Witepsol W 45[(R)]) | 1 680,0 mg |
| | 1 700,0 mg |

Herstellungsverfahren:

Die feinpulverisierte Wirksubstanz wird in der geschmolzenen und auf 40 °C abgekühlten Zäpfchenmasse suspendiert. Man gießt die Masse bei 37 °C in leicht vorgekühlte Zäpfchenformen aus.
Zäpfchengewicht 1,7 g

Beispiel V

Tropfen mit 5,11-Dihydro-11-[[[2-[2-[(dipropylamino)methyl]-1-piperidinyl]ethyl]amino]carbonyl]-6H-pyrido-[2,3-b][1,4]benzodiazepin-6-on-methansulfonat

Zusammensetzung:

100 ml Tropflösung enthalten:

| | | |
|---|---|---|
| p-Hydroxybenzoesäuremethylester | | 0,035 g |
| p-Hydroxybenzoesäurepropylester | | 0,015 g |
| Anisöl | | 0,05 g |
| Menthol | | 0,06 g |
| Ethanol rein | | 10,0 g |
| Wirkstoff | | 0,5 g |
| Natriumcyclamat | | 1,0 g |
| Glycerin | | 15,0 g |
| Dest. Wasser | ad | 100,0 ml |

Herstellungsverfahren:

Die Wirksubstanz und Natriumcyclamat werden in ca. 70 ml Wasser gelöst und Glycerin zugefügt. Man

löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

Beispiel VI

Suppositorien mit 20 mg 5,11-Dihydro-11-[[3-[3-(1-piperidinyl)propyl]-1-piperidinyl]carbonyl]-6H-pyrido[2,3-b][1,4]-benzodiazepin-6-on

Zusammensetzung:

1 Zäpfchen enthält:

| | |
|---|---|
| Wirkstoff | 20,0 mg |
| Zäpfchenmasse (z.B. Witepsol W 45(R)) | 1 680,0 mg |
| | 1 700,0 mg |

Herstellungsverfahren:

Die feinpulverisierte Wirksubstanz wird in der geschmolzenen und auf 40° C abgekühlten Zäpfchenmasse suspendiert. Man gießt die Masse bei 37° C in leicht vorgekühlte Zäpfchenformen aus.
Zäpfchengewicht 1,7 g

Beispiel VII

Tabletten mit 5 mg 4,9-Dihydro-3-methyl-4-[[4-[3-(1-piperidinyl)propyl]-1-piperidinyl]acetyl]-10H-thieno[3,4-b][1,5]-benzodiazepin-10-on

Zusammensetzung:

1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45° C getrocknet, nochmals durch obiges Sieb gerieben, mit Magnesiumstearat vermischt und zu Tabletten verpreßt.
Tablettengewicht: 220 mg

Stempel: 9 mm

Beispiel VIII

Tropfen mit 5,11-Dihydro-11-[6-(1-piperidinyl)-1-oxohexyl]-6H-pyrido[2,3-b][1,4]benzodiazepin-6-on

Zusammensetzung:

100 ml Tropflösung enthalten:

| | | |
|---|---|---|
| p-Hydroxybenzoesäuremethylester | | 0,035 g |
| p-Hydroxybenzoesäurepropylester | | 0,015 g |
| Anisöl | | 0,05 g |
| Menthol | | 0,06 g |
| Ethanol rein | | 10,0 g |
| Wirkstoff | | 0,5 g |
| Natriumcyclamat | | 1,0 g |
| Glycerin | | 15,0 g |
| Dest. Wasser | ad | 100,0 ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

Beispiel IX

Suppositorien mit 50 mg 4,9-Dihydro-3-methyl-4-[1-oxo-6-(hexahydro-1H-1-azepinyl)-4-hexinyl]-10H-thieno-[3,4-b]-[1,5]benzodiazepin-10-on

Zusammensetzung:

1 Zäpfchen enthält:

| | |
|---|---|
| Wirkstoff | 50,0 mg |
| Zäpfchenmasse (z.B. Witepsol W 45(R)) | 1 695,0 mg |
| | 1 745,0 mg |

Herstellungsverfahren:

Die feinpulverisierte Wirksubstanz wird in der geschmolzenen und auf 40° C abgekühlten Zäpfchenmasse suspendiert. Man gießt die Masse bei 37° C in leicht vorgekühlte Zäpfchenformen aus.
Zäpfchengewicht 1,745 g

Beispiel X

Tabletten mit 5 mg 11-[4-[3-[(Diethylamino)methyl]-4-morpholinyl]-1-oxobutyl]-5,11-dihydro-6H-pyrido[2,3-b][1,4]-benzodiazepin-6-on

Zusammensetzung:

1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemisch und mit obigem Schleim durch ein Sieb der Maschenweite 1,5 mm granuliert. Das Granulat wird bei 45° C getrocknet, nochmals durch obiges Sieb gerieben, mit Magnesiumstearat vermischt und zu Tabletten verpreßt.
Tablettengewicht: 220 mg
Stempel: 9 mm

Beispiel XI

Tropfen mit 5,11-Dihydro-11-[[[2-(1-methyl-2-pyrrolidinyl)ethyl]methylamino]acetyl]-6H-pyrido[2,3-b][1,4]-benzodiazepin-6-on

Zusammensetzung:

100 ml Tropflösung enthalten:

| | | |
|---|---|---|
| p-Hydroxybenzoesäuremethylester | | 0,035 g |
| p-Hydroxybenzoesäurepropylester | | 0,015 g |
| Anisöl | | 0,05 g |
| Menthol | | 0,06 g |
| Ethanol rein | | 10,0 g |
| Wirkstoff | | 0,5 g |
| Natriumcyclamat | | 1,0 g |
| Glycerin | | 15,0 g |
| Dest. Wasser | ad | 100,0 ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

Beispiel XII

Tabletten mit 5 mg 5,11-Dihydro-11-[[[2-(1-methyl-hexahydro-1H-2-azepinyl)ethyl]methylamino]acetyl]-6H-pyrido[2,3-b]-[1,4]benzodiazepin-6-on

Zusammensetzung:

1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45° C getrocknet, nochmals durch obiges Sieb gerieben, mit Magnesiumstearat vermischt und zu Tabletten verpreßt.
Tablettengewicht: 220 mg
Stempel: 9 mm

Beispiel XIII

Tabletten mit 5 mg 11-[[4-[4-(Diethylamino)butyl]-1-piperidinyl]acetyl]-5,11-dihydro-6H-pyrido[2,3-b][1,4]-benzodiazepin-6-on

Zusammensetzung:

1 Tablette enthält:

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzukker und die restliche Kartoffelstärke werden gemischt und mit obigem Schleim durch ein Sieb der Maschenweite 1,5 mm granuliert. Das Granulat wird bei 45 °C getrocknet, nochmals durch obiges Sieb gerieben, mit Magnesiumstearat vermischt und zu Tabletten verpreßt.
Tablettengewicht: 220 mg
Stempel: 9 mm

**Ansprüche**

1.) Verwendung von kondensierten Diazepinonen der allgemeinen Formel Ia

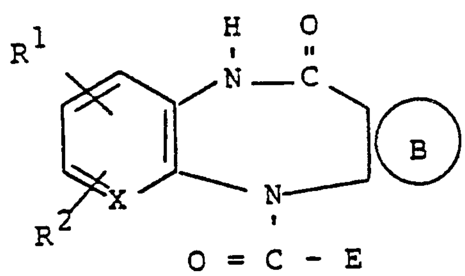

(Ia)

in der ] Ⓑ einen der zweiwertigen Reste

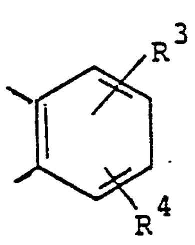 , 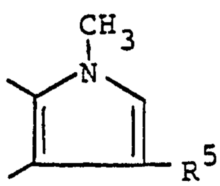 , 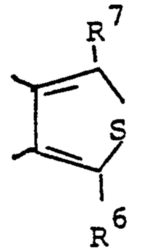 , 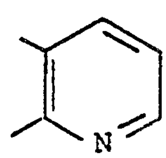 ,

(S) (T) (U) (V)

bedeutet und
X, $R^1$ bis $R^7$ und E die folgenden Bedeutungen besitzen:
a.) E ist eine Gruppe der Formel II

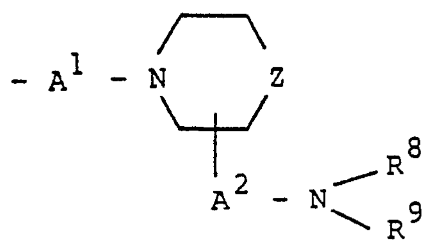

(II)

sofern X die =CH-Gruppe ist und ] Ⓑ für einen der zweiwertigen Reste (S), (T) oder (U) steht oder, im Falle, daß ] Ⓑ den ortho-Phenylenrest darstellt, X auch ein Stickstoffatom bedeutet,
wobei in den Formeln Ia und II
$A^1$ einen Alkylenrest mit 1 bis 2 Kohlenstoffatomen, $A^2$ entweder einen Alkylenrest mit 1 oder 2 Kohlenstoffatomen, sofern er sich in der 2-Stellung zum Stickstoff des gesättigten, heterocyclischen Ringes befindet oder, wenn er sich in der 3- oder 4-Stellung befindet, eine Einfachbindung oder die Methylengruppe bedeuten, und
Z entweder eine Einfachbindung oder ein Sauerstoffatom oder die Methylen- oder 1,2-Ethylengruppe darstellt,
$R^1$ eine Alkylgruppe mit 1 bis 4 C-Atomen, ein Chloratom oder ein Wasserstoffatom;
$R^2$ ein Wasserstoffatom oder eine Methylgruppe,
$R^3$ und $R^4$ und jeweils ein Wasserstoffatom, ein Fluor-, Chlor- oder Bromatom oder eine Alkylgruppe mit 1 bis 4 C-Atomen bedeuten,
$R^5$ ein Wasserstoff- oder Chloratom oder die Methylgruppe,
$R^6$ ein Wasserstoff- oder Chloratom oder einen Alkylrest mit 1 bis 4 C-Atomen darstellen,
$R^7$ ein Wasserstoffatom oder einen Alkylrest mit 1 bis 4 C-Atomen,
$R^8$ einen verzweigten oder unverzweigten Alkylrest mit 1 bis 3 C-Atomen,
$R^9$ einen verzweigten oder unverzweigten Alkylrest mit 1 bis 7 C-Atomen darstellen, wobei gegebenenfalls der Alkylrest noch an seinem 2. bis 7. Kohlenstoffatom durch eine Hydroxygruppe substituiert sein kann, wobei $R^9$ aber auch einen Cycloalkyl- oder Cycloalkylmethylrest mit 3 bis 7 C-Atomen im Ring, der gegebenenfalls noch durch eine Hydroxygruppe substituiert sein kann, bedeuten kann, und im Falle, daß $R^1$ bis $R^4$ nicht alle gleichzeitig Wasserstoffatome bedeuten sondern wenigstens einer dieser Reste eine der anderen oben angegebenen Bedeutungen einnimmt, $R^8$ und $R^9$ lediglich gleiche oder verschiedene Alkylreste mit 1 bis 6 C-Atomen, $A^1$ die Methylengruppe und X das Stickstoffatom bedeuten; wobei in der Gruppe E $R^8$ und $R^9$ aber auch zusammen mit dem dazwischenliegenden Stickstoffatom einen 4- bis 7-gliedrigen gesättigten, monocyclischen heterocyclischen Ring bilden können, der gegebenenfalls durch ein Sauerstoffatom oder durch die $N-CH_3$-Gruppe unterbrochen sein kann,
b.) E ist eine Gruppe der Formel III

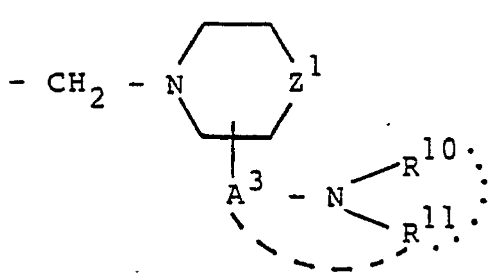

(III)

sofern in der Formel Ia
X eine =CH-Gruppe oder ein Stickstoffatom,
$R^1$ und $R^2$ Wasserstoffatome und

] Ⓑ die zweiwertigen Reste (S), (T), (U) und (V) mit den oben angegebenen Bedeutungen für die Reste $R^3$ bis $R^7$ darstellen,

wobei in der Formel III

$A^3$ einen geradkettigen oder verzweigten, gesättigten Alkylenrest mit 3 bis 7 Kohlenstoffatomen, der auch durch ein Sauerstoff- oder Schwefelatom oder die Gruppe $>NR^{12}$, in der $R^{12}$ für eine Alkylgruppe mit 1 bis 3 C-Atomen steht, unterbrochen sein kann, darstellt,

$Z^1$ eine Einfachbindung, ein Sauerstoff- oder Schwefelatom, die Methylen- oder 1,2-Ethylengruppe bedeutet,

$R^{10}$ einen verzweigten oder unverzweigten Alkylrest mit 1 bis 7 Kohlenstoffatomen, einen Cycloalkyl- oder (Cycloalkyl)alkylrest mit insgesamt bis zu 8 Kohlenstoffatomen, eine gegebenenfalls mit einem Fluor-, Chlor- oder Bromatom und/oder einer Methyl-, Methoxy- oder Trifluormethylgruppe substituierte Aralkylgruppe mit bis zu 9 Kohlenstoffatomen, eine aliphatische Acylgruppe mit bis zu 7 Kohlenstoffatomen oder eine gegebenenfalls durch ein Fluor-, Chlor- oder Bromatom und/oder eine Methyl-, Methoxy- oder Trifluormethylgruppe substituierte Benzoylgruppe bedeutet,

$R^{11}$ eine verzweigte oder unverzweigte Alkylgruppe mit 1 bis 6 Kohlenstoffatomen darstellt wobei $R^{11}$ aber auch ein Wasserstoffatom sein kann, wenn $R^{10}$ die vorerwähnte aliphatische Acylgruppe oder gegebenenfalls substituierte Benzoylgruppe darstellt,

$R^{10}$ und $R^{11}$ aber auch zusammen mit dem dazwischenliegenden Stickstoffatom einen gesättigten, monocyclischen 5-, 6- oder 7-gliedrigen Ring bilden können, der gegebenenfalls noch durch eine Aminocarbonyl-, Dimethylaminocarbonyl- oder Diethylaminocarbonylgruppe substituiert und/oder durch ein Sauerstoffatom unterbrochen sein kann,

$R^{11}$ aber auch mit einem Kohlenstoffatom der $A^3$-kette in der Weise verknüpft sein kann, daß zusammen mit der Gruppe $NR^{12}$ ein gesättigter 5-, 6- oder 7-gliedriger heterocyclischer Ring entsteht,

c.) E ist eine Grupppe der Formel IV

$-A^4 - CH_2 - C \equiv C - CH_2 - A^5$ (IV)

sofern in der Formel Ia

X ein Stickstoffatom, ] Ⓑ den ortho-Phenylenrest und $R^1$ bis $R^4$ Wasserstoffatome bedeuten, wobei in der Formel IV

$A^4$ die Gruppen $H-\underset{|}{\overset{|}{C}}-R^{13}$, $\underset{|}{\overset{|}{N}}-R^{13}$ oder -0-, und

$A^5$ die Gruppen

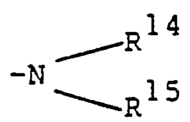

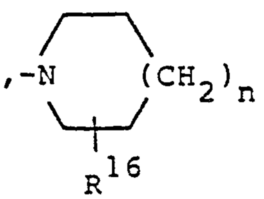

oder

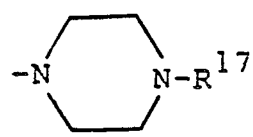

bedeuten, worin

$R^{13}$ ein Wasserstoffatom oder eine Alkylgruppe mit 1 bis 3 Kohlenstoffatomen,

$R^{14}$ und $R^{15}$, die gleich oder voneinander verschieden sein können, ein Wasserstoffatom, einen Alkylrest mit 1 bis 3 Kohlenstoffatomen, einen Phenylalkylrest mit insgesamt 7 bis 9 Kohlenstoffatomen, einen gegebenenfalls durch eine Hydroxygruppe substituierten 5- bis 7-gliedrigen Cycloalkylrest,

n die Zahlen 0, 1 oder 2,

$R^{16}$ ein Wasserstoffatom, eine Hydroxygruppe, eine Alkylgruppe mit 1 bis 3 Kohlenstoffatomen oder eine Gruppe der Formel

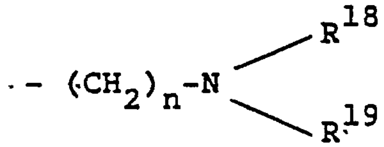

,

worin n wie oben definiert ist und $R^{18}$ und $R^{19}$ einen Alkylrest mit 1 bis 3 Kohlenstoffatomen bedeuten und

$R^{17}$ eine gerade oder verzweigte Alkylgruppe mit 1 bis 3 Kohlenstoffatomen oder eine Phenylalkylgruppe mit insgesamt 7 bis 9 Kohlenstoffatomen darstellen,
d.) E ist eine Gruppe der Formel V

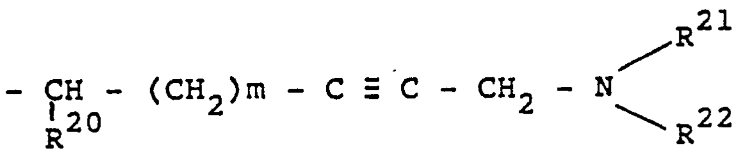

(V)

sofern in der Formel Ia
X eine =CH-Gruppe und ] Ⓑ den zweiwertigen Rest (U) bedeuten, worin $R^6$ und $R^7$ wie oben definiert sind, $R^6$ aber zusätzlich noch ein Fluor- oder Bromatom sein kann und $R^1$ und $R^2$ Wasserstoffatome sind,
wobei in der Formel V
$R^{20}$ ein Wasserstoffatom oder eine Alkylgruppe mit 1 bis 3 Kohlenstoffatomen,
$R^{21}$ ein Wasserstoffatom oder eine Alkylgruppe mit 1 bis 4 Kohlenstoffatomen,
$R^{22}$ eine gegebenenfalls durch eine Phenylgruppe substituierte Alkylgruppe mit 1 bis 3 Kohlenstoffatomen oder eine gegebenenfalls durch eine Hydroxygruppe substituierte 5- bis 7-gliedrige Cycloalkylgruppe oder
$R^{21}$ und $R^{22}$ zusammen mit dem dazwischenliegenden Stickstoffatom einen 5- bis 7-gliedrigen gegebenenfalls durch einen Dialkylaminoalkylrest, eine Hydroxygruppe oder einen Alkylrest substituierten gesättigten monocyclischen Ring oder den 1-Piperazinylrest, der in 4-Stellung durch eine Alkyl-, Phenylalkyl- oder Phenylgruppe substituiert ist, wobei jeweils die vorstehend erwähnten Alkylteile 1 bis 3 Kohlenstoffatome enthalten können, und
m die Zahlen 1, 2 oder 3 darstellen,
e.) E ist eine Gruppe der Formel VI

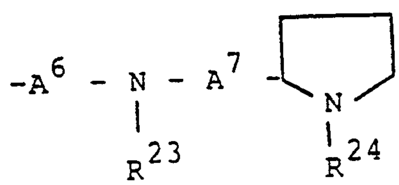

(VI)

sofern in der Formel Ia
X eine =CH-Gruppe oder ein Stickstoffatom, ] Ⓑ die zweiwertigen Reste (S), (T), (U), (V) und zusätzlich den zweiwertigen Rest

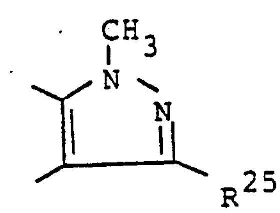

(W)

darstellen, in welchen die Reste $R^1$ bis $R^7$ wie oben definiert sind, aber $R^6$ noch zusätzlich ein Fluor- oder Bromatom sein kann, und $R^{25}$ ein Wasserstoffatom oder die Methylgruppe bedeutet,
wobei in der Formel VI
$A^6$ und $A^7$ geradkettige, gesättigte Alkylenreste mit 1 bis 4 Kohlenstoffatomen,
$R^{23}$ und $R^{24}$ Wasserstoffatome oder verzweigte oder unverzweigte Alkylreste mit 1 bis 4 Kohlenstoffatomen oder Cycloalkylreste mit 4 bis 7 Kohlenstoffatomen, die gegebenenfalls noch durch eine Hydroxygruppe substituiert sein können, bedeuten,
wobei in dem Fall, daß ] Ⓑ den zweiwertigen Rest (T) darstellt und $R^5$ ein Wasserstoffatom ist, $R^1$ kein

Chloratom sein kann und in dem Fall, daß ] Ⓑ den zweiwertigen Rest (V) bedeutet, X kein Stickstoffatom ist,

f.) E ist eine Gruppe der Formel VII

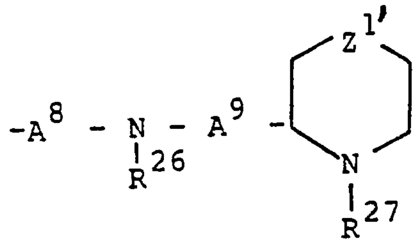

(VII)

sofern in der Formel Ia

X eine =CH-Gruppe, ein Stickstoffatom oder, zusätzlich, eine =C-Cl-Gruppe, ] Ⓑ einen der zweiwertigen Reste (S), (T), (U) oder (V) bedeuten, wobei ] Ⓑ nur dann den zweiwertigen Rest (V) bedeutet, wenn X die =CH-Gruppe oder =C-Cl-Grupppe darstellt, und wobei $R^1$ und $R^2$ Wasserstoffatome bedeuten und $R^3$ bis $R^7$ wie oben definiert sind, und $R^6$ zusätzlich noch die Bedeutung eines Fluor- oder Bromatoms haben kann,

wobei in der Formel VII

$A^8$, und $A^9$ geradkettige oder verzweigte gesättigte Alkylenreste mit 1 bis 4 Kohlenstoffatomen,

$Z^1$ ein Sauerstoffatom oder eine Alkylenkette mit 1 bis 3 Kohlenstoffatomen

$R^{25}$ einen verzweigten oder unverzweigten Alkylrest mit 1 bis 7 Kohlenstoffatomen, einen Cycloalkylrest oder (Cycloalkyl)alkylrest mit insgesamt bis zu 8 Kohlenstoffatomen oder ein Wasserstoffatom und

$R^{27}$ einen verzweigten oder unverzweigten Alkylrest mit 1 bis 6 Kohlenstoffatomen oder eine Cycloalkylgruppe mit bis zu 7 Kohlenstoffatomen bedeuten,

von deren Isomeren und physiologisch verträglichen Salzen mit anorganischen oder organischen Säuren und

von kondensierten Diazepinonen der allgemeinen Formel Ib

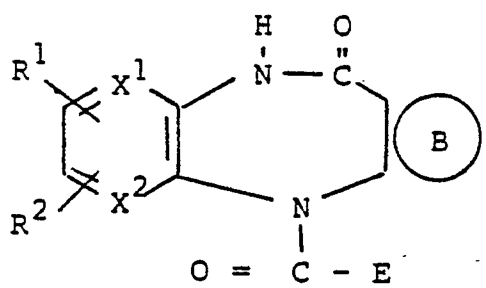

(Ib)

in der die Substituenten wie folgt definiert sind

] Ⓑ bedeutet einen der zweiwertigen Reste

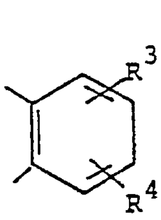
,
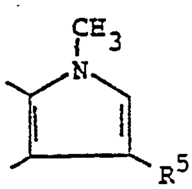
,
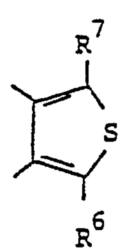
,
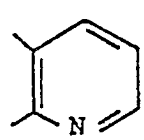
oder
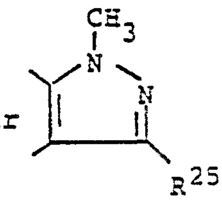

(S) (T) (U) (V) (W)

$X^1$ und $X^2$ eine =CH-Gruppe oder, sofern ] Ⓑ die Bedeutungen der oben genannten Reste (S), (U) oder (W) annimmt, können beide oder nur $X^1$ oder nur $X^2$ auch ein Stickstoffatom sein,

$R^1$ bis $R^7$ und $R^{25}$ sind wie im Falle der Formel Ia definiert, wobei $R^6$ zusätzlich auch ein Fluor- oder Bromatom sein kann,
und E bedeutet
a.) eine Gruppe der Formel VIII

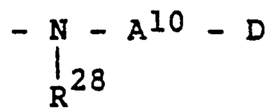

(VIII)

in der
$R^{28}$ ein Wasserstoffatom oder eine Methylgruppe,
D die Gruppen

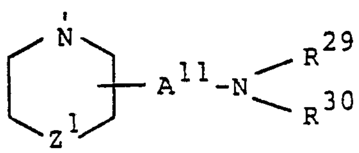

,

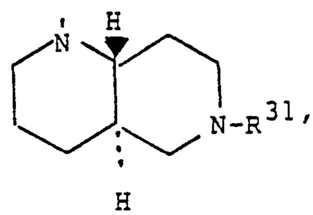

,

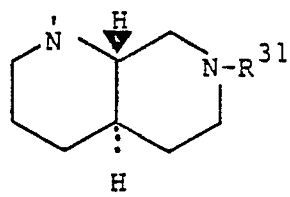

,

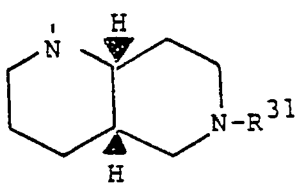

und

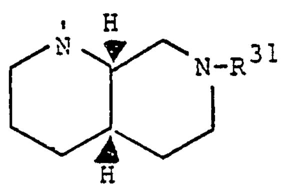

und
$A^{10}$ einen geradkettigen oder verzweigten Alkylenrest mit 2 bis 7 Kohlenstoffatomen darstellen, wobei in den Gruppen D
$Z^1$ wie oben für die Formel Ia definiert ist,
$A^{11}$ einen geradkettigen oder verzweigten gesättigten Alkylenrest mit 1 bis 5 Kohlenstoffatomen oder, wenn $A^{11}$ sich in 3-Stellung zum Stickstoff des gesättigten heterocyclischen Ringes befindet, auch eine Einfachbindung darstellt,
$R^{29}$ einen verzweigten oder unverzweigten Alkylrest mit 1 bis 4 Kohlenstoffatomen,
$R^{30}$ einen verzweigten oder unverzweigten Alkylrest mit 1 bis 7 Kohlenstoffatomen, der gegebenenfalls noch an seinem 2. bis 7. Kohlenstoffatom durch eine Hydroxygruppe substituiert sein kann, des weiteren einen Cycloalkyl- oder einen Cycloalkylmethylrest mit 3 bis 7 Kohlenstoffatomen im Ring, wobei der Cycloalkylring gegebenenfalls noch durch eine Hydroxygruppe substituiert sein kann, bedeuten, wobei $R^{29}$ und $R^{30}$ aber auch zusammen mit dem dazwischenliegenden Stickstoffatom einen 4- bis 7-gliedrigen gesättigten, monocyclischen, heterocyclischen Ring bilden können, der gegebenenfalls durch ein Sauerstoffatom oder durch die Gruppe $>N-CH_3$ unterbrochen sein kann, und
$R^{31}$ einen verzweigten oder unverzweigten Alkylrest mit 1 bis 6 Kohlenstoffatomen darstellt,
b.) eine Gruppe der Formel IX

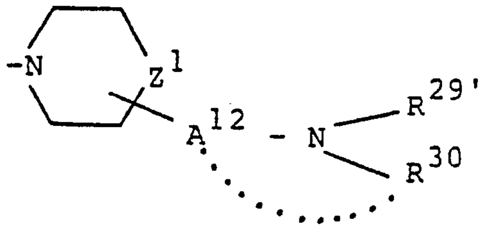

(IX)

worin

$Z^1$ wie oben definiert ist,

$A^{12}$ einen geradkettigen oder verzweigten gesättigten Alkylenrest mit 2 bis 7 Kohlenstoffatomen, der auch durch ein Sauerstoff- oder Schwefelatom oder durch die Methylimino-oder Ethyliminogruppe unterbrochen sein kann, bedeutet und

$R^{29}$ und $R^{30}$ die vorstehend genannten Bedeutungen innehaben, wobei sich $R^{29'}$ von $R^{29}$ dadurch unterscheidet, daß $R^{29}$ zusätzlich noch die Bedeutung einer Benzylgruppe einnehmen kann: $R^{30}$ kann aber zusätzlich auch mit $A^{12}$ über eine Alkylenbrücke in der Weise verknüpft sein, daß zusammen mit der Gruppe $-NR^{29}$ ein gesättigtes, 5-, 6- oder 7-gliedriges heterocyclisches Ringsystem entsteht,

c.) eine Gruppe der Formel X

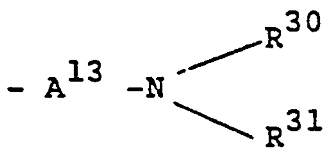

(X)

in der

$R^{30}$ eine Alkylgruppe mit 1 bis 3 Kohlenstoffatomen,

$R^{31}$ eine gegebenenfalls ab dem 2. Kohlenstoffatom durch eine Hydroxygruppe substituierte geradkettige oder verzweigte Alkylgruppe mit 1 bis 8 Kohlenstoffatomen, eine Cycloalkyl- oder Cycloalkylmethylgruppe, in welcher der Cycloalkylteil jeweils 3 bis 8 Kohlenstoffatome enthalten kann und durch eine Methyl- oder Hydroxygruppe substituiert sein kann, eine Phenylalkylgruppe, in welcher der Alkylteil 1 bis 3 Kohlenstoffatome enthalten kann und der Phenylrest durch ein Halogenatom, eine Methyl-, Methoxy- oder Trifluormethylgruppe mono- oder disubstituiert sein kann, wobei die Substituenten des Phenylkerns gleich oder verschieden sein können bedeuten, oder

$R^{30}$ und $R^{31}$ zusammen mit dem dazwischenliegenden Stickstoffatom einen 6- bis 8-gliedrigen gesättigten monocyclischen Ring bilden, in welchem eine Methylengruppe mit der Maßgabe, daß mindestens zwei Kohlenstoffatome zwischen dieser Methylengruppe und dem bereits vorhandenen Stickstoffatom liegen, durch ein Sauerstoffatom oder durch eine gegebenenfalls durch eine Alkyl-, Phenylalkyl- oder Phenylgruppe substituierte Iminogruppe, in welcher der Alkylteil jeweils 1 bis 3 Kohlenstoffatome enthalten kann und der Phenylkern jeweils durch ein Halogenatom, eine Methyl-, Methoxy- oder Trifluormethylgruppe mono- oder disubstituiert sein kann, wobei die Substituenten gleich oder verschieden sein können, ersetzt sein kann,

$A^{13}$ eine gegebenenfalls eine Doppelbindung enthaltende Alkylengruppe mit 5 bis 8 Kohlenstoffatomen darstellt mit der Maßgabe, daß

mindestens 5 Kohlenstoffatome zwischen der semicyclischen Carbonylgruppe und dem Stickstoffatom der

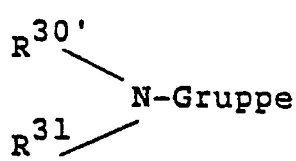

liegen,
d.) eine Gruppe der Formel XI

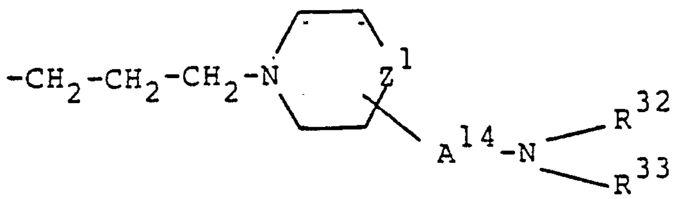

(XI)

worin $Z^1$ wie oben definiert ist,
$A^{14}$ einen geradkettigen, gesättigten Alkylenrest mit 1 bis 6 Kohlenstoffatomen,
$R^{32}$ und $R^{33}$ gleiche oder voneinander verschiedene Alkylreste mit bis zu 6 Kohlenstoffatomen bedeuten, die zusammen mit dem dazwischenliegenden Stickstoffatom einen 4- bis 7-gliedrigen, gesättigten, monocyclischen, heteroaliphatischen Ring, der gegebenenfalls durch ein Stickstoffatom oder durch die >N-$CH_3$ Gruppe unterbrochen sein kann, bilden können,
von deren diastereomeren und enantiomeren Formen, und von deren physiologisch verträglichen Salzen mit anorganischen oder organischen Säuren zur Therapie von Erkrankungen des Zentralnervensystems.

2.) Verwendung von kondensierten Diazepinonen der allgemeinen Formeln Ia und Ib gemäß Anspruch 1 zur Bekämpfung des Morbus Alzheimer.

3.) Verwendung von kondensierten Diazepinonen der allgemeinen Formeln Ia und Ib gemäß Anspruch 1 zur Bekämpfung des Morbus Parkinson.

4.) Verwendung von kondensierten Diazepinonen der allgemeinen Formeln Ia und Ib gemäß Anspruch 1 als Antiemetika.

5.) Verwendung von kondensierten Diazepinonen der allgemeinen Formeln Ia und Ib gemäß Anspruch 1 zur Förderung der cerebralen Durchblutung in der Geriatrie und zur Bekämpfung der Migräne.

6.) Verwendung von kondensierten Diazepinonen der allgemeinen Formeln Ia und Ib gemäß Anspruch 1 zur Herstellung von Arzneimitteln mit den in den Ansprüchen 1 bis 5 genannten Wirkungen.